# EUROPEAN PATENT SPECIFICATION

(11) **EP 1 683 512 B1**
(45) Date of publication and mention of the grant of the patent: **26.03.2014**
(21) Application number: 04030563.3
(22) Date of filing: 23.12.2004
(51) Int. Cl.: A61K 8/362, A61K 8/365, A61K 8/49, A61Q 5/02, A61Q 5/12

(54) **Hair conditioning composition containing flavone derivatives**
Flavonoiden enthaltende Haarpflegezusammensetzung
Composition de soin capillaire contenant des derivés de flavone

(43) Date of publication of application: 26.07.2006
(73) Proprietor: Kao Germany GmbH, 64297 Darmstadt (DE)
(72) Inventor: Molenda, Michael, 60598 Frankfurt (DE); Lateulere, Magali, 64297 Darmstadt (DE); Golinski, Frank Dr., 64372 Ober-Ramstadt (DE); Lichtl, Rixa Dr., 64287 Darmstadt (DE)

(56) References cited:
- EP-A- 0 680 744
- EP-A- 1 174 112
- EP-A1- 0 743 311
- EP-A1- 0 884 044
- EP-A1- 0 978 272
- DE-A1- 19 928 495
- FR-A- 2 578 422
- US-A1- 2002 013 481

## Description

The present invention is related to a conditioning composition for hair comprising flavone derivatives. The conditioning composition of the present invention can be in the form of a shampoo, cleansing - conditioning composition, or in the form of a conditioner used after washing hair with cleansing compositions.

Flavone derivatives comprising hair conditioning compositions are known in the literature. For example, EP 680 744 is on the used of flavone derivatives in cosmetic composition as a protection agent for the mechanical properties of hair. Furthermore, DE 33 20 539 A1 is on hair cosmetic compositions comprising flavonol derivatives.

Deposition of flavone derivatives onto hair has been found to be very weak from up until now known hair cosmetic compositions and consequently either low in effect is observed or very high concentration of the actives are used for achieving the desired effects. Therefore, there is a great need for improvement in the deposition of flavone derivatives onto hair to increase their effectiveness.

US 2002 / 0013481 A1 discloses use of flavones for protecting ascorbic acid and/or ascorbyl compounds from oxidation. The document does not relate to enhancement of deposition of flavones onto hair.

EP 1 174 112 A2 is on acidic hair conditioning compositions comprising fatty alcohol, cationic surfactant, organic solvend and acidic compounds, The document does not disclose anything on deposition of flavones onto hair.

EP 680 744 A1 is on the use of flavones for protecting hair. The disclosed compositions comprising flavones and organic solvent in water are primarily for leave in usage. pH ranges are disclosed but is not linked with the deposition of flavones onto hair.

The objective of the current invention is to find out a composition from which the flavone derivatives as formulated show high deposition onto hair compared to the compositions disclosed in the literature. It should be noted that the present invention is mainly concerned rinse off formulations, however, the application of the compositions of the current invention as leave in product is not excluded.

It has surprisingly been found out that deposition of flavone derivatives selected from t-flavanone, quercetin and morin is enhanced from a composition comprising at the same time at least one hair conditioning agent, at least one organic solvent and at least one hydroxycarboxylic acid and/or dicarboxylic acid and having a pH in the range of 2.0 to 4.0. The conditioning compositions show additionally optimum performance in hair shine improving, making hair excellently manageable and soft. The compositions of the present invention also improve combability, volume and body of hair. After using the compositions of present invention, hair feels nicer and more natural when touching. The effects mentioned are more pronounced on repeated usage.

EP 1174112 discloses hair cosmetic compositions comprising organic acid, organic solvent, cationic surfactant and higher alcohol and having pH in the range of 2 to 6 for improving hair shine. Additionally, WO 2004/047777 discloses leave-in compositions for hair comprising malic and lactic acids and organic solvents for improving shine, setting and touch feeling. Both documents are silent on flavone derivatives and especially improving deposition of flavone derivatives and/or any other active ingredient form hair conditioning compositions onto hair.

The pH of the compositions according to the present invention is in the range of 2.0 to 4.0, more preferably 2.5 to 3.8.

pH of the compositions is adjusted with hydroxycarboxylic acids and/or dicarboxylic acids. In those cases where selected hydroxycarboxylic acid and/or dicarboxylic acid concentration is not enough to reach the selected pH, other organic and inorganic acids may also be used to adjust pH to the required value. The hydroxycarboxylic acids useful in the compositions of the present invention are lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid.

Compositions according to invention comprise at least one hydroxycarboxylic acid and/or dicarboxylic acid. Especially preferred hydroxycarboxylic acids are the lactic and malic acids. Malic acid is also a dicarboxy acid. The most preferred hydroxycarboxylic acid is the malic acid.

Total hydroxycarboxylic acid and/or dicarboxylic acid concentration in the composition of the present invention varies in the range form 0.1 to 5% by weight, preferably 0.25 to 4% by weight and more preferably 0.5 to 3% by weight calculated to the total composition. In a further preferred embodiment of the invention, the compositions of the present invention comprise at least 0.5% malic acid.

Synthesis of t-flavanone is disclosed in EP 743 311 B1. The flavone derivatives are incorporated into the compositions of the present invention either as commercially available pure (the word pure should not be taken as 100% purity and should be understood as flavone derivative enriched raw materials containing other substances) raw material and as well as in the form of natural extracts such as green tea extract, gingko extract, etc.

Concentration of the flavone derivatives in the compositions of the present invention is typically in the range of 0.01% to 2%, by weight, preferably 0.01 to 1.5% and more preferably 0.05 to 1.0% by weight, calculated to total composition.

Conditioning composition comprises organic solvents such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylene carbonate, propylene glycol, polypropylene glycols, ethylene glycol monoethylether, ethylene glycol monobutyl ether, ethylene glycol monophenyl ether, 1-phenylethylalcohol, 2-phenylethylalcohol, o-methoxyphenol. The most preferred ones are benzyl alcohol, ethanol, benzyloxyethanol, propylene glycol and polypropylene glycol. Concentration of organic solvents should not exceed 10% by weight, preferably in the range of 0.1 to 7.5% by weight calculated to total composition in compositions designed for cleansing and conditioner / treatment in emulsion form. Higher organic solvent concentrations may be suitable for the preparation such as hair conditioning solution applied by a spraying device. In such case organic solvent concentration can be as high as 50% by weight.

The compositions of the present invention can be either a conditioning -cleansing composition or a conditioning composition typically used after use of cleansing compositions

The composition of the present invention comprises hair-conditioning agents in any type of composition. Conditioning agents can be selected from oily substances, non-ionic substances, cationic amphiphilic ingredients, cationic polymers or their mixtures.

Oily substances are selected from such as silicone oils, either volatile or nonvolatile, natural and synthetic oils. Among silicone oils those can be added to the compositions include dimethicone, dimethiconol, polydimethylsiloxane, DC fluid ranges from Dow Corning, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁ CO (O CH₂ CH₂)ₙ OH

or

R₁ CO (O CH₂ CH₂)ₙ O OC R₂

where R₁ and R₂ are independent from each other saturated, unsaturated or branched or non-branched alkyl chain with 7 to 21 C atoms and n is typically 2 - 100.

In one of the preferred from of the present invention, conditioning compositions comprise at least one cationic conditioning agent. Cationic conditioning agents are cationic polymers and cationic surfactants. Suitable cationic polymers are those of best known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

It has further been found out that especially those of cationic cellulose type polymers known as Polymer JR type from Amerchol such as Polyquaternium 10 or cationic guar gum known with trade name Jaguar from Rh6ne-Poulenc and chemically for example Guar hydroxypropyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, polyquaternium 6 and polyquaternium 7.

The cationic polymers also include the quaternized products of graft polymers from organopolysiloxanes and polyethyl oxazolines described in EP-A 524 612 and EP-A 640 643.

Cationic surfactants suitable for the present invention are according to the general formula 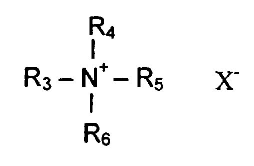 where R₃ is a saturated or unsaturated, branched or non-branched alkyl chain with 8-22 C atoms or

R₇ CO NH (CH₂)ₙ

where R₇ is saturated or unsaturated, branched or non-branched alkyl chain with 7-21 C atoms and n has value of 1 - 4,
or

R₈ CO O (CH₂)ₙ

where R₈ is saturated or unsaturated, branched or non-branched alkyl chain with 7-21 C atoms and n has value of 1 - 4, and
R₄ is hydrogen or unsaturated or saturated, branched or non-branched alkyl chain with 1 - 4 C atoms or

R₇ CO NH (CH₂)ₙ

or

R₈ CO O (CH₂)ₙ

where R₇, R₈ and n are same as above.

R₅ and R₆ are hydrogen or lower alkyl chain with 1 to 4 carbon atoms, and X is anion such as chloride, bromide, methosulfate.

Typical examples of those ingredients are cetyltrimethyl ammonium chloride, steartrimonium chloride, behentrimonium chloride, stearamidopropyl trimonuim chloride, dioleoylethyl dimethyl ammonium methosulfate, dioleoylethyl hydroxyethylmonium methosulfate.

Amido amines may as well be used as a conditioning cationic surfactant in the compositions of the present invention. Typical non-limiting example is stearamidopropylamine known with a trade name Tego Amid S18 from Degussa. The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those conditioners of cationic polymers, silicon oil and derivatives and cationic surfactants can be 0.01 - 10% by weight, preferably 0.01 - 7.5% by weight, more preferably 0.05 - 5% and most preferably 0.1 - 3% by weight calculated to the total composition. It should be noted that especially non-cleansing conditioning type of the products contain higher concentrations (of the above mentioned concentrations) of the cationic surfactants which at the same time, if desired, can be emulsifying agent. In cleansing and conditioning type of preparations, concentration of cationic surfactants is towards the lower end of the above mentioned concentration, approximately below 1 % by weight.

Conditioning compositions of the present invention is a cleansing composition (cleansing-conditioning composition). Cleansing conditioning compositions of the present invention comprise at least one surfactant selected from anionic, nonionic and/or amphoteric or zwitterionic surfactants at a concentration range of 1 to 50%, preferably 5 to 40% and more preferably 5 to 30%, and most preferably 5 to 25% by weight, calculated to the total composition.

In one of the preferred embodiment of the present invention cleansing conditioning composition of the present invention, comprises at least one anionic, at least one nonionic surfactant. More preferably, the compositions further comprise additionally at least one amphoteric surfactant.

Anionic surfactants suitable within the scope of the invention are preferably present in an amount from 1 to about 30%, preferably 2 to 20% and most preferably 2 - 15%, by weight, calculated to the total composition.

These are anionic surfactants of the sulfate, sulfonate, carboxylate and alkyl phosphate type, especially, of course, those customarily used in shampoo compositions, for example, the known C₁₀-C₁₈-alkyl sulfates, and in particular the respective ether sulfates, for example, C₁₂₋C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates constituting mild, skin-compatible detergents.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.
Suitable surfactants of the carboxylate type are alkyl polyether carboxylic acids and the salts thereof of the formula

R₉ - (C₂H₄O)ₙ- O - CH₂COOX,

wherein R₉ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 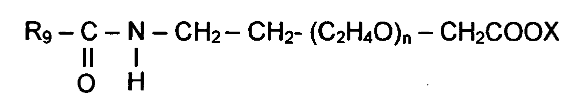 wherein R₉ and X have the above meanings, and n is in particular a number from 1 to 10, preferably 2.5 to 5.

Such products have been known for some time and are on the market, for example, under the trade name "AKYPO® " and "AKYPO-SOFT® ".

Also useful are C₈-C₂₀-acyl isethionates, alone or in admixture with other anionic surfactants, as well as sulfofatty acids and the esters thereof.

It is also possible to use mixtures of several anionic surfactants, for example an ether sulfate and a polyether carboxylic acid or alkyl amidoether carboxylic acid.

An overview of the anionic surfactants used in liquid body cleansing compositions can furthermore be found in the monography of K. Schrader, "Grundlagen und Rezepturen der Kosmetika", 2nd Ed.(1989, Hüthig Buchverlag), pp. 595-600 and pp. 683 to 691.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂₋C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

Further surfactants in the shampoo compositions according to the invention are nonionic surfactants in admixture with anionic surfactants.

These are described in Schrader, I.c., on pages 600-601 and pp. 694-695. Especially suited are alkyl polyglucosides of the general formula

R₁₀-O-(R₁₁O)ₙ-Zₓ,

wherein R₁₀ is an alkyl group with 8 to 18 carbon atoms, R₁₁ is an ethylene or propylene group, Z is a saccharide group with 5 to 6 carbon atoms, n is a number from 0 to 10 and x is a number between 1 and 5.

These alkyl polyglucosides have recently become known in particular as excellent skin-compatible, foam improving agents in liquid detergents and body cleansing compositions, and are present in an amount from about 1 % to 15 %, in particular from 1 % to 10 % by weight, calculated to the total composition.

Mixtures of anionic surfactants and alkyl polyglucosides as well as the use thereof in liquid body cleansing compositions are already known, for example, from EP-A 70 074. The alkyl polyglucosides disclosed therein are basically also suited within the scope of the present invention; as well as the mixtures of sulfosuccinates and alkyl polyglucosides disclosed in EP-A 358 216.

Further nonionic surfactant components are, for example, long-chain fatty acid mono- and dialkanolamides, such as coco fatty acid monoethanolamide and myristic fatty acid monoethanolamide, which can also be used as foam enhancers, preferably in amounts from about 1 % to about 5 % by weight.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates.

Further nonionic surfactants useful in the compositions according to invention are C₁₀-C₂₂-fatty alcohol ethoxylates at a concentration of 0.5 to 10%, preferably 0.5 to 5% by weight, calculated to total composition. Especially suited are C₁₀-C₂₂₋fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":
The average degree of ethoxylation thereby ranges between about 2.5 and about 25, preferably about 10 and about 20.

As further surfactant component, in another preferred form of invention, the compositions especially those of cleansing and conditioning according to the invention comprise at least one amphoteric or zwitterionic surfactants in an amount from 0.5 % to 15 %, preferably from 1 % to 10 %, more preferably from 1 % to 5 %by weight, calculated to the total composition. It has especially been found out that addition of zwitterionic or amphoteric surfactants enhances foam feeling in terms of creaminess, foam volume and as well as skin compatibility is improved. For achieving milder formulations anionic surfactant, especially of sulphate types, to amphoteric surfactant ratio should be in the range of 10:1 to 1:1, preferably 5:1 to 1:1.

Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structure 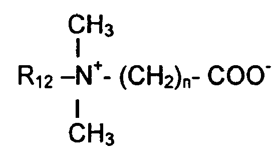 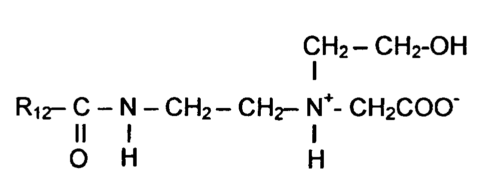 wherein R₁₂ is a C₈-C₁₈-alkyl group and n is 1 to 3;
sulfobetaines of the structure 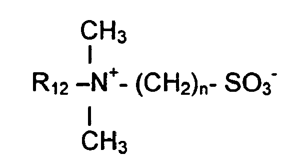 wherein R₁₀ and n are same as above;
and amidoalkyl betaines of the structure 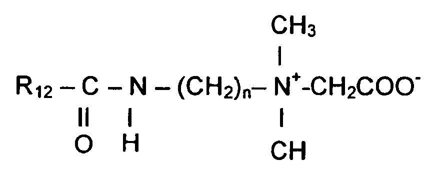 wherein R₁₂ and n are same as above.

Solubilizers may be added to the compositions, in particular cleansing compositions, especially when oily substances are chosen as conditioning agents and fragrance oils with highly lipophilic properties. Typical solubilizers may be hydrogenated castor oil known with the trade mark Cremophor RH series from BASF. It should be noted that as well the surfactant mixture can be a good solubilizer for fragrance oils. Typical concentration of the solubilizers can be in the range of 0.01 - 2% by weight, preferably 0.1 - 1% by weight, calculated to total composition.

Further conditioning additives are hair conditioning and/or styling polymers into either cleansing or conditioning type. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol® ", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful in conditioning composition of any type of the present invention. They are incorporated alone or in admixture with at least one additional cationic, nonionic or anionic polymer, particularly copolymers of N-octyl acrylamide, (meth)acrylic acid and tert.-butyl aminoethyl methacrylate of the type "Amphomer® "; copolymers from methacryl oylethyl betaine and alkyl - methacrylates of the type "Yukaformer® ", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl - aminoalkyl (meth)acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199, are applicable.

Conditioning and cleansing composition of the present invention can be transparent as well as pearly. Transparency of the composition is judged by naked eye in a transparent shampoo bottle with a thickness not more than 5 cm. In the case a transparent appearance is wished, the following ingredients are not essential. Pearl-shiny appearance is achieved with those dispersed in cleansing conditioning compositions in crystalline form, i.e. so called pearl-shine or pearlizing agents. The preferred once are PEG-3 distearate and ethylene glycol distearate. The concentration of those can typically be from 0.1 to 3%, preferably 0.5 to 2% by weight, calculated to the total composition. These compounds are preferably added to the compositions in admixture with anionic, nonionic and/or amphoteric surfactants. Such kind of mixtures is available commercially.

Hair cleansing conditioning compositions of the present invention can be in the form of conventional liquid thickened shampoo, as well in the form of ready to use foam, delivered either from a pump-foamer or from an aerosol bottle. In the case that an aerosol foam preparation is preferred, propellant gas must be added to the formulation. The suitable propellant gasses are carbondioxide, dimethylether and alkanes such as butane propane or their mixtures.

Conditioning compositions of the present invention can be in the form of emulsions, solutions, gels and dispersions. In the case that solutions and/or gels forms are preferred the appearance can be either with a transparent or opaque. As a product form, foam is as well suited when packed into a pressurized can or delivered through a pump-foamer (non-aerosol). In the case that an aerosol foam preparation is preferred, propellant gas must be added to the formulation. The suitable propellant gasses are carbondioxide, dimethylether and alkanes such as butane, propane, isobutane or their mixtures.

The emulsion type of conditioners comprise additionally at least one fatty alcohol of the following formula

R₁₃-OH

where R₁₃ is a saturated or unsaturated, branched or non-branched alkyl chain with 8 - 24 C atoms. Concentration of fatty alcohols is usually less than 20%, preferably less than 15%, more preferably in the range of 1 to 10% by weight calculated to total composition. Typical examples to the most useful fatty alcohols are myristyl alcohol, palmityl alcohol, cetyl alcohol, stearyl alcohol, behenyl alcohol and their mixtures. As a mixed fatty alcohol the mostly used one is the cetearyl alcohol as well preferred in the compositions of the present invention.

The conditioning compositions of any type may contain active ingredients selected from UV filters, moisturisers, sequestering agents, and natural ingredients.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

The UV filters are those oil and water soluble ones for the purpose of protecting hair. In other words, anionic and nonionic, oil soluble, UV filters are suitably used in the compositions of the present invention. Suitable UV-absorbing substances is are: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxy- benzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher. The amount of the UV-absorber ranges typically from about 0.01 % to 2.5%, more preferably from 0.05 % to 1 % by weight, calculated to the total composition.

Natural plant extracts may be incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderbeny, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc. Suitable trade products are, for example, the various "Extrapon® " products, "Herbasol^{R} ", "Sedaplant^{R}" and "Hexaplant^{R}". Extracts and the preparation thereof are also described in "Hagers Handbuch der pharmazeutischen Praxis", 4th Ed..

The viscosity of the conditioning shampoo compositions according to the invention is in the range of 500 and about 20,000 mPa.s at 20°C, preferably 1,000 to 10,000, in particular 1,500 to 8,000 mPa.s at 20°C, measured with Höppler viscosimeter.

Viscosity of shampoo compositions can be adjusted with known viscosity enhancers. The preferred ones are monoglycerides such as glyceryl laurate, PEG-55 propyleneglycol oleate and PEG-18 glyceryl oleate/cocoate known with the trade names Antil^{R} 141 and 171, respectively and PEG-160 sorbitan triisostearate known with a trade name Rheodol^{R}. It should be noted that in the case that a composition are delivered in the form of a foam from a pump-foamer and/or aerosol can, those compositions should not be thickened and have a viscosity value not more than 500 mPa.s, more preferably 250 mPa.s measured as mentioned above at room temperature.

Viscosity of the non-cleansing conditioning composition may not be more than 50,000 mPa.s at 20°C measured with Brookfield Rheometer at a shear rate of 10 sec⁻¹.

It should especially be noted that the effects of the inventive compositions become more and more visible after repeated usage.

The following examples are to illustrate the invention, but not to limit. The compositions according to the invention are prepared by mixing the individual components in water, whereby it is also possible to use pre-mixtures of various ingredients.

### Example 1

### Determination of flavone derivatives deposition onto hair

Beached buffalo hair tresses (weighing approximately 2g) were soaked into preparations (100 ml) for 5 min and afterwards the swatches were rinsed off with tap water and were dried with an hair dryer. The hair swatches were than soaked into ethanol (96%) for the extraction of flavone derivative deposited. The same was carried out with a blanc, untreated hair tress. The concentration of flavone derivative was than determined spectrophotometrically by measuring absorbance values of the sample solutions at 320, 360 and 375 nm for t-Flavonone, Quercetin and Morin, respectively. The amount deposited was calculated by using a calibration curve made in the same solution, extract of hair. The results are expressed as µg flavone derivative / g hair.

The following solutions were tested for demonstrating the effect of pH and organic solvent on deposition of t-Flavonon, Quercetin and Morin onto hair.

| | Concentration % by weight | |
|---|---|---|
| | Solution A | Solution C |
| t-Flavanone or Quercetin or Morin | 0.3 | 0.3 |
| Cetrimoniumchloride | 0.5 | 0.5 |
| Malic acid | - | 1.0 |
| Lactic acid | - | 0.5 |
| Benzylalcohol | - | 2.5 |
| Ethanol | - | 5.0 |
| Water | to 100 | to 100 |
| pH | 5.5 | 3.4 |

**Table I: The results of flavone derivatives deposition**

| | Flavone derivative deposited µg/g hair | |
|---|---|---|
| | Solution A | Solution C |
| t-Flavanone | 35 | 56 |
| Quercetin | 47 | 170 |
| Morin | 314 | 2140 |

From the above results it is obvious that by reducing pH of the same composition with addition of malic and lactic acids and addition of organic solvents, the deposition of flavone derivatives enhanced dramatically.

Similar results are observed with the composition below.

### Example 2

**Shampoo composition**

| | |
|---|---|
| Sodium lauryl ether sulfate | 11.0 (% by wt.) |
| Coco glucoside | 4.0 |
| Cocoamidopropyl betaine | 1.5 |
| Cationic polymer (Polyquaternium-10) | 0.2 |
| Benzylalcohol | 0.25 |
| Perfume, preservative | q.s |
| PEG-60-hydrogenated castor oil | 0.5 |
| PEG-18 Glyceryl cocoate/oleate | 2.0 |
| t-flavanone | 0.3 |
| Malic acid | 1.00 |
| Water | ad 100.0 |

The pH of the composition is 3.2.

### Example 3

**Shampoo composition**

| | |
|---|---|
| Sodium lauryl ether carboxylate (10EO) | 5.0 (% by wt.) |
| Coco glucoside | 5.0 |
| Cocoamidopropyl betaine | 5.0 |
| Cationic polymer (Polyquaternium-7) | 0.2 |
| Benzylalcohol | 0.5 |
| Perfume, preservative | q.s. |
| PEG-60-hydrogenated castor oil | 0.5 |
| PEG-18 Glyceryl cocoate/oleate | 1.0 |
| Quercetin | 0.3 |
| Lactic acid | 0.40 |
| Malic acid | 0.50 |
| Water | ad 100.0 |

The pH of the composition is 3.5.

### Example 4

**Shampoo composition**

| | |
|---|---|
| Coco glucoside | 5.0 |
| Cocoamidopropyl betaine | 5.0 |
| Laureth-16 | 2.0 |
| Lauroyl glutamate | 3.0 |
| Cationic polymer (Polyquaternium-11) | 0.5 |
| PEG-3 distearate | 0.8 |
| Benzylalcohol | 0.5 |
| Perfume, preservative | q.s. |
| PEG-18 Glyceryl cocoate/oleate | 0.80 |
| Morin | 0.1 |
| Malic acid | 0.75 |
| Water | ad 100.0 |

The pH of the composition is 3.6.

### Example 5

**Foam shampoo**

| | |
|---|---|
| Coco glucoside | 5.0 |
| Cocoamidopropyl betaine | 6.0 |
| Sodium laureth sulfate | 4.0 |
| Cationic polymer (Polyquaternium-11) | 0.5 |
| Benzylalcohol | 0.5 |
| Perfume, preservative | q.s. |
| Benzphenone-4 | 0.2 |
| t-flavanone | 0.2 |
| Malic acid | 0.75 |
| Lactic acid | 0.25 |
| Water | ad 100.0 |

The pH of the composition is 3.4. The above composition is a very low viscosity composition, in any case a viscosity lower than 500 mPa.s measured at ambient temperature and with Höppler viscosimeter, confectioned into a pump-foamer as purchased from the company Air-Spray - Germany and showed excellent conditioning and shine effect

Similarly and aerosol foam shampoo was prepared by confectioning the above composition at a weight ratio of 90/10 - composition/propellant- using propane-butane mixture as a propellant. The foam shampoo so obtained showed excellent cleansing and brightening and shine effects.

### Example 6

**Hair treatment composition rinse-off**

| | |
|---|---|
| Cetylstearylalcohol | 5.0 (% by weight) |
| Stearyltrimethylammoniumchlorid | 2.0 |
| Benzylalchol | 2.5 |
| t-flavanone | 0.15 |
| Benzophenone-4 | 0.2 |
| Fragrance, preservative | q.s. |
| Malic acid | 1.0 |
| Wasser | ad 100.0 |

The pH of the composition is 3.0.

### Example 7

**Foam conditioner**

| | |
|---|---|
| Quaternium-80 | 0.2 (Gew.-%) |
| Polyquaternium-11 | 0.7 |
| PEG-160-hydrogenated ricinus oil | 0,5 |
| Fragrance, preservative | q.s. |
| t-flavanone | 0.1 |
| Benzophenone-4 | 0.2 |
| Malic acid | 0.5 |
| Lactic acid | 0.2 |
| Wasser | ad 100.0 |

pH of the composition is adjusted to 3.4. The composition is suitable for leave-in and rinse off. In leave-in application, amount (whole composition for as a conditioner applied onto hair) used is obviously less than in the case of a rinse of application. The composition is packed into an aerosol can with 90/10 ratio, by weight, liquid composition to propellant. As propellant propane, butane mixture is used.

### Example 8

**Conditioner**

| | |
|---|---|
| Cetylstearylalcohol | 5.0 (% by weight) |
| Cetrimoniumchloride | 1.0 |
| Panthenol | 0,4 |
| Dimethicone | 0.75 |
| Hydroxypropyl Guar Hydroxypropyltrimonium | |
| Chloride | 1.0 |
| t.flavanone | 0.2 |
| Avocado extract | 0.5 |
| Fragrance, preservative | q.s. |
| Malic acid | 0.8 |
| Wasser | ad 100.0 |

The pH of the composition is 3.6.

### Example 9

**Conditioner**

| | |
|---|---|
| Cetylstearylalcohol | 5.0 (% by weight) |
| Dioleoylethyldimethylammonium ethosulfate | 1.0 |
| Ceteareth 20 | 1.0 |
| Panthenol | 0,4 |
| Dimethicone | 0.75 |
| Hydroxypropyl Guar Hydroxypropyltrimonium | |
| Chloride | 1.0 |
| Morin | 0.2 |
| Avocado extract | 0.5 |
| Fragrance, preservative | q.s. |
| Malic acid | 0.8 |
| Wasser | ad 100.0 |

The pH of the composition is 3.6.

## Claims

1. Conditioning composition for hair **characterized in that**, it comprises at least one hair conditioning agent, at least one flavone derivative selected from t-flavanone, quercetin and morin, at least one organic solvent and at least one hydroxycarboxylic acid and/or dicarboxylic acid and has a pH in the range of 2.0 to 4.0.

2. Composition according to claim 1 **characterized in that** at least one flavone is present in the compositions at a concentration of 0.001 to 2% by weight, calculated to total composition.

3. Composition according any of the preceding claims **characterized in that** concentration of one or more hydroxycarboxylic acid and/or dicarboxylic acid is in the range of 0.1 to 5% by weight calculated to the total composition.

4. Composition according any of the preceding claims **characterized in that** it comprises malic acid and/or lactic acid as hydroxycarboxylic acid.

5. Composition according any of the preceding claims **characterized in that** it comprises hydroxycarboxylic acid and/or dicarboxylic acid at concentration of 0.5 to 5% by weight with the condition that it comprises malic acid at a concentration of not less than 0.5% by weight calculated to total composition.

6. Composition according any of the preceding claims **characterized in that** it comprises only malic acid as a hydroxycarboxylic acid and/or dicarboxylic acid.

7. Composition according any of the preceding claims **characterized in that** it comprises as organic solvents benzyl alcohol and/or ethanol and/or benzyloxyethanol and/or polypropylene glycol.

8. Composition according any of the preceding claims **characterized in that** it comprises as conditioning agent at least one cationic polymer and/or at least one cationic surfactant.

9. Composition according to any of the preceding claims **characterized in that** composition is a cleansing and conditioning composition and comprises at least one surfactant selected from anionic, nonionic and amphoteric or zwitterionic surfactants at a concentration of 1 to 50% by weight calculated to the total composition.

10. Composition according to claim 9 **characterized in that** it comprises at least one anionic surfactant and at least one non-ionic surfactant.

11. Composition according to claims 9 and 10 **characterized in that** it comprises additionally at least one amphoteric surfactant.

12. Composition according to claims 1 to 8 **characterized in that** composition is a conditioning composition in emulsion form and comprises at least one fatty alcohol.

13. Composition according any of the preceding claims **characterized in that** it comprises at least one UV filter.

14. Use of composition according to any of the preceding claims for enhancing deposition of flavone derivatives onto hair.

## Patentansprüche

1. Konditionierende Zusammensetzung für die Haare, **dadurch gekennzeichnet, dass** sie mindestens ein Haarkonditionierungsmittel, mindestens ein Flavonderivat, ausgesucht aus t-Flavon, Querzetin und Morin, mindestens ein organisches Lösungsmittel und mindestens eine Hydroxycarboxylsäure und/oder Dicarboxylsäure enthält und einen pH-Wert im Bereich von 2,0 bis 4,0 aufweist.

2. Zusammensetzung nach Anspruch 1, **dadurch gekennzeichnet, dass** sie mindestens ein Flavon zu 0,001 bis 2 Gew.% enthält, und zwar bezogen auf die Gesamtzusammensetzung.

3. Zusammensetzung nach einem der vorgehenden Anspruch, **dadurch gekennzeichnet, dass** die Konzentration einer oder mehrerer Hydroxycarboxylsäure(n) und/oder Dicarboxylsäure(n) in einem Bereich von 0,1 bis 5 Gew.% liegt, und zwar bezogen auf die Gesamtzusammensetzung.

4. Zusammensetzung nach einem der vorgehenden Anspruch, **dadurch gekennzeichnet, dass** sie Apfelsäure und/oder Milchsäure als Hydroxycarboxylsäure enthält.

5. Zusammensetzung nach einem der vorgehenden Anspruch, **dadurch gekennzeichnet, dass** sie Hydroxycarboxylsäure und/oder Dicarboxylsäure in einem Bereich von 0,5 bis 5 Gew.% enthält, mit der Bedingung, dass sie, bezogen auf die Gesamtzusammensetzung, Apfelsäure in einer Konzentration von nicht weniger als 0,5 Gew.% enthält.

6. Zusammensetzung nach einem der vorgehenden Anspruch, **dadurch gekennzeichnet, dass** sie als Hydroxycarboxylsäure und/oder Dicarboxylsäure nur Apfelsäure enthält.

7. Zusammensetzung nach einem der vorgehenden Anspruch, **dadurch gekennzeichnet, dass** sie als organische Lösungsmittel Benzylalkohol und/oder Ethanol und/oder Benzylalkohol und/oder Polypropylenglycol enthält.

8. Zusammensetzung nach einem der vorgehenden Anspruch, **dadurch gekennzeichnet, dass** sie mindestens ein kationisches Polymer und/oder ein kationisches Tensid als Haarkonditionierungsmittel enthält.

9. Zusammensetzung nach einem der vorgehenden Anspruch, **dadurch gekennzeichnet, dass** die Zusammensetzung eine Reinigungs- und Konditionierungszusammensetzung ist und mindestens ein Tensid, ausgesucht aus den anionischen, nichtionischen und amphoterischen oder zwitterionischen Tensiden, enthält, und zwar in einer Konzentration von 1 bis 50 Gew.%, bezogen auf die Gesamtzusammensetzung

10. Zusammensetzung nach Anspruch 9, **dadurch gekennzeichnet, dass** sie mindestens ein anionisches Tensid und mindestens ein nichtionisches Tensid enthält.

11. Zusammensetzung nach Anspruch 9 und 10, **dadurch gekennzeichnet, dass** sie zusätzlich mindestens ein amphoterisches Tensid enthält.

12. Zusammensetzung nach einem der Ansprüche 1 bis 8, **dadurch gekennzeichnet, dass** diese Zusammensetzung eine konditionierende Zusammensetzung in Emulsionsform ist und mindestens ein Fettalkohol enthält.

13. Zusammensetzung nach einem der vorgehenden Anspruch, **dadurch gekennzeichnet, dass** sie mindestens einen UV-Filter enthält.

14. Verwendung der Zusammensetzung nach einem der vorgehenden Anspruch, zur Verbesserung der Aufbringung von Flavonderivaten auf das Haar.

## Revendications

1. Composition de conditionnement pour les cheveux **caractérisée par le fait qu'**elle comprend au moins un agent de conditionnement pour les cheveux, au moins un dérivé de flavone choisi parmi la t-flavanone, la quercétine et la morine, au moins un solvant organique et au moins un acide hydroxycarboxylique et/ou dicarboxylique et ayant une valeur pH allant de 2,0 à 4,0

2. Composition, selon la revendication 1, **caractérisée par le fait qu'**au moins une flavone est présente dans les compositions à une concentration en poids de 0,001 à 2%, calculée par rapport à la composition totale

3. Composition, selon l'une quelconque des revendications précédentes, **caractérisée par le fait que** la concentration en poids de l'un ou plusieurs des acides hydroxycarboxyliques et/ou dicarboxyliques est comprise entre 0,1 et 5%, calculée par rapport à la composition totale.

4. Composition, selon l'une quelconque des revendications précédentes, **caractérisée par le fait qu'**elle contient de l'acide malique et/ou lactique comme acide hydroxycarboxylique.

5. Composition, selon l'une quelconque des revendications précédentes, **caractérisée par le fait qu'**elle comprend de l'acide hydroxycarboxylique et/ou dicarboxylique à une concentration en poids de 0,5 à 5% à la condition qu'elle comprenne l'acide malique à une concentration en poids d'au moins 0,5% calculée par rapport à la composition totale.

6. Composition, selon l'une quelconque des revendications précédentes, **caractérisée par le fait qu'**elle comprend uniquement de l'acide malique comme acide hydroxycarboxylique et/ou dicarboxylique.

7. Composition, selon l'une quelconque des revendications précédentes, **caractérisée par le fait qu'**elle comprend comme solvants organiques de l'alcool benzylique et/ou d'éthanol et/ou du benzyloxyéthanol et/ou polypropylène glycol.

8. Composition, selon l'une quelconque des revendications précédentes, **caractérisée par le fait qu'**elle comprend comme agent de conditionnement au moins un polymère cationique et/ou au moins un surfactant cationique.

9. Composition, selon l'une quelconque des revendications précédentes, **caractérisée par le fait que** la composition est une composition nettoyante et de conditionnement et qu'elle comprend au moins un surfactant choisi parmi les surfactants anioniques, non-ioniques et amphotères ou zwitterioniques à une concentration en poids de 1 à 50% calculée par rapport à la composition totale.

10. Composition, selon la revendication 9, **caractérisée par le fait qu'**elle comprend au moins un surfactant anionique et au moins un surfactant non-ionique.

11. Composition, selon les revendications 9 et 10, **caractérisée par le fait qu'**elle comprend en plus au moins un surfactant amphotère.

12. Composition, selon les revendications 1 à 8, **caractérisée par le fait que** la composition est une composition de conditionnement sous forme d'émulsion et comprend au moins un alcool gras.

13. Composition, selon l'une quelconque des revendications précédentes, **caractérisée par le fait qu'**elle comprend au moins un filtre UV.

14. Utilisation d'une composition, selon l'une quelconque des revendications précédentes, pour améliorer la déposition des dérivés de flavone sur les cheveux.
